# FASCICULE DE BREVET EUROPEEN

(11) **EP 0 926 127 B1**
(45) Date de publication et mention de la délivrance du brevet: **04.10.2001**
(21) Numéro de dépôt: 98403160.9
(22) Date de dépôt: 15.12.1998
(51) Int. Cl.: C07C 67/303, C07C 69/716, C07C 69/738, C07C 45/33, C07C 49/12

(54) **Procédé de préparation de composés alfa, bêta-dicarbonylés acycliques**
Verfahren zum Herstellen von acyclischen alpha,beta-Dicarbonylverbindungen
Process for the preparation of acyclic alpha,beta-dicarbonyl compounds

(30) Priorité: 26.12.1997 FR 9716588
(43) Date de publication de la demande: 30.06.1999
(73) Titulaire: Clariant (France), 92800 Puteaux (FR)
(72) Inventeur: Vallejos, Jean-Claude, 13008 Marseille (FR); Capelle, Nicolas, 30000 Nîmes (FR); Arzoumanian, Henri, 13400 Aubagne (FR)
(74) Mandataire: Rinuy, Santarelli

(56) Documents cités:
- J. COSSY ET AL.: "Oxidative Cleavage of 2-Substituted Cycloalkane-1,3-diones and of Cyclic beta-Ketoesters by Copper Perchlorate/Oxygen" TETRAHEDRON LETTERS., vol. 35, no. 33, 15 août 1994, pages 6089-6092, XP002076550 OXFORD GB
- AHMED ATLAMSANI ET AL.: "Synthesis of 5- and 6-Oxoalkanoic Acids by Copper(II)-Catalized Oxidative Cleavage of Cycloalkanones with Dioxygen" SYNTHESIS., no. 1, janvier 1993, pages 79-81, XP000335853 STUTTGART DE
- ZONG-XING SI ET AL: "A Facile General Route to alpha-Keto Esters and alpha-Diketones" SYNTHESIS., no. 6, juin 1990, pages 509-510, XP000142401 STUTTGART DE
- ITO S. ET AL.: "Ferric Salt Catalyzed Oxydation of Cycloalkanones to Oxo Esters by Molecular Oxygen" JOURNAL OF ORGANIC CHEMISTRY., vol. 48, no. 7, 8 avril 1983, pages 1133-1135, XP002076551 EASTON US

## Description

La présente invention concerne un procédé de préparation de composés α,β -dicarbonylés acycliques.

Il existe dans la littérature de nombreuses voies de synthèse des α-cétoesters, des α-cétoacides et des α,β-dicétones.

On peut citer par exemple :
- Kovacs, L. *Recl. Trav*. *Chim*. *Pays-Bas* **112,** 471-496 (1996),
- Cooper, A.J.L. et al, *Chem*.*Rev*. **83,** 321-358 (1983),
- Weinstock, L.M. et al, *Synth. Comm.* **11,** 943 (1981),
- Wislicenus *Ber* **20**., 592 (1887),
- Friedman, L. et Kosower, E. *Organic Syntheses*, Coll. Vol.lll, 510,
- Sohda, T. et al, *Chem*. *Pharm. Bull.* **30,** 3601 (1982),
- Inokushi, T. et al, *Chem*. *Lett.* **8**, 1411-1414 (1994),
- Muckawa, H. et al,. *Chem*. *Lett.* **6,** 1017-1020 (1994),
- Bouveault, L. et Locquin, *R. Bull*. *Soc. Chim. Fr,* **31,** 1049,1055, 1061, 1143 (1904),
- Locquin, R. *Bull Soc. Chim.* Fr. **31**, 1068,1147(1904),
- Si, Z et al, *Synthesis* **6**, 509-510 (1990).

Mais aucune des synthèses décrites ne permet d'envisager une exploitation industrielle.

En effet, les procédés décrits exigent soit des matières premières rarement disponibles sur le marché, soit des réactifs coûteux, soit des conditions opératoires non transposables industriellement ou sont des procédés polluants.

Or la demanderesse a découvert avec étonnement qu'il était possible d'obtenir économiquement et avec de bons rendements des composés α,β-dicarbonylés acycliques en faisant réagir, au sein d'un solvant de réaction convenable, un composé de départ adéquat avec de l'oxygène moléculaire en présence de faibles quantités d'un catalyseur convenable.

C'est pourquoi la présente demande a pour objet un procédé de préparation de composés α,β -dicarbonylés acycliques de formule (I) 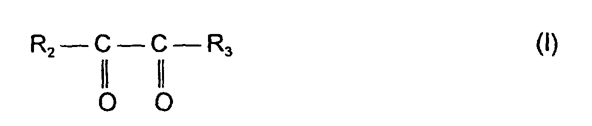 dans laquelle R₂ représente un radical alkyl renfermant de 1 à 6 atomes de carbone ou un radical éthoxy, ou un radical phényl ou un radical benzyl pouvant être diversement substitué sur le cycle, notamment par des substituants alkyl renfermant de 1 à 6 atomes de carbone, par des substituants alkoxy renfermant de 1 à 4 atomes de carbone ou par des subsituants halogènes ou un radical ester carboxylique renfermant de 2 à 5 atomes de carbone et R₃ représente un radical alkyl renfermant de 1 à 4 atomes de carbone, ou un radical alkoxy renfermant de 1 à 4 atomes de carbone ou un radical ester carboxylique renfermant de 2 à 5 atomes de carbone, caractérisé par le fait que l'on fait réagir dans un solvant de type nitrile un composé α,γ dicarbonylé acyclique de formule (II) 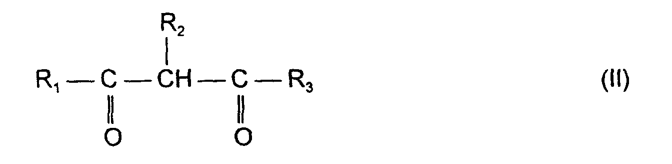 dans laquelle R₁ représente un radical alkyl renfermant de 1 à 6 atomes de carbone, ou un radical éthoxy ou un radical α-cétocarboxylate d'alkyle renfermant de 3 à 6 atomes de carbone ou un radical ester carboxylique renfermant de 2 à 5 atomes de carbone et R₂ et R₃ ont les mêmes significations que précédemment, avec de l'oxygène moléculaire en présence de nitrate de cuivre^{II} ou de chlorure ferrique comme catalyseur.

Les composés α,β-dicarbonylés acycliques de formule I concernent essentiellement des α-cétoesters qui permettent d'accéder aux α-cétoacides et leurs dérivés qui sont des molécules très intéressantes à la fois pour leur utilisation dans les systèmes biologiques et en synthèse organique. En effet, certains α-cétoacides sont utilisés dans le traitement de malades dialysés, c'est le cas par exemple pour l'acide méthyléthylpyruvique, l'acide diméthylpyruvique ou l'acide isopropylpyruvique. De plus ils sont d'un grand intérêt comme intermédiaire pour de nombreuses synthèses telles que celles de médicaments, enzymes d'inhibition, α-hydroxyacides, c-nucléosides, produits naturels, ainsi que pour la synthèse d'un grand nombre d'hétérocycles.

Ils peuvent être également des α-β-dicétones, composés très intéressants pour leur utilisation en synthèse organique.

Comme catalyseur convenable, il a été découvert de façon étonnante que parmi les nombreux sels de cuivre de fer, de cobalt et de manganèse testés, seuls le nitrate de cuivre" et le chlorure ferrique donnaient des rendements de synthèse intéressants et exploitables industriellement.

En outre comme solvant de réaction convenable, il a été découvert de façon étonnante qu'avec les catalyseurs retenus, seuls les solvants de type nitrile, particulièrement aliphatique ou aromatique, permettaient d'obtenir des rendements de synthèse intéressants et exploitables industriellement, les solvants de type aliphatique donnant les meilleurs rendements.

Le procédé selon la présente invention permet l'obtention de façon économique et avec de bons rendements, des composés α,β -dicarbonylés acycliques de formule (I) ci-dessus.

Dans la formule I, dans la formule Il et dans ce qui suit, le terme radical alkyl renfermant de 1 à 6 atomes de carbone représente par exemple un radical méthyl, éthyl, propyl, butyl, hexyl, cyclohexyl, de préférence un radical méthyl ou éthyl et particulièrement un radical éthyl.

Dans la formule I et dans ce qui suit, le radical phényl ou le radical benzyl peut être diversement substitué sur le cycle par un ou plusieurs substituants, de préférence par 3 ou 2 ou 1 substituants alkyl renfermant de 1 à 6 atomes de carbone, alkoxy renfermant de 1 à 4 atomes de carbone ou halogène, de préférence chlore ou brome et se trouve préférentiellement sans substitution.

Dans la formule I et dans ce qui suit, le terme radical alkoxy renfermant de 1 à 4 atomes de carbone représente par exemple, un radical butoxy ou propyloxy ou éthoxy ou méthoxy, de préférence un radical éthoxy ou méthoxy et particulièrement éthoxy.

Dans la formule I, dans la formule Il et dans ce qui suit, le terme radical ester carboxylique renfermant de 2 à 5 atomes de carbone représente par exemple un carboxylate de butyle ou de propyle ou d'éthyle ou de méthyle, de préférence un radical carboxylate d'éthyle ou de méthyle et particulièrement un carboxylate d'éthyle.

Dans la formule Il et dans ce qui suit, le terme radical α-cétocarboxylate d'alkyle renfermant de 3 à 6 atomes de carbone représente par exemple un radical α-cétocarboxylate de butyle ou de propyle ou d'éthyle ou de méthyle, de préférence un radial α-cétocarboxylate d'éthyle ou de méthyle et particulièrement un radical α-cétocarboxylate d'éthyle.

Dans des conditions préférentielles de mise en oeuvre du procédé de l'invention, le solvant de réaction de type nitrile sera de type nitrile aliphatique, tout particulièrement l'acétonitrile.

Selon d'autres conditions préférentielles de mise en oeuvre du procédé ci-dessus, la quantité de nitrate de cuivre" ou de chlorure ferrique sera comprise entre 0,1 équivalent molaire et 0,001 équivalent molaire et plus particulièrement entre 0,05 et 0,01 équivalent molaire par rapport au substrat de départ de formule (II).

Selon d'autres conditions préférentielles de mise en oeuvre du procédé ci-dessus, la réaction sera effectuée en milieu saturé en oxygène réalisé par un barbotage d'oxygène ou d'air dans le milieu réactionnel.

Selon encore d'autres conditions préférentielles de mise en oeuvre du procédé ci-dessus, la réaction sera effectuée en milieu saturé en oxygène réalisé sous pression d'oxygène de 10,13 10⁴ Pa à 50,65 10⁴ Pa (1 à 5 atmosphères).

Toujours dans des conditions préférentielles de mise en oeuvre du procédé ci-dessus, l'oxygène utilisé provient d'un débit d'air ou d'une pression d'air.

Le procédé selon l'invention sera mis en oeuvre plus particulièrement à température ambiante mais pourra être effectué à plus haute température si on veut réduire le temps de réaction ou la Quantité de catalyseur utilisé.

Précisons encore que le procédé selon l'invention est favorisé par une plus grande dilution du substrat dans le solvant de type nitrile mais que, pour une utilisation industrielle du procédé, on préférera travailler à une concentration pondérale de l'ordre de 20 à 25 % du substrat dans le solvant de type nitrile.

Le procédé selon l'invention, est avantageusement mis en oeuvre en mélangeant dans un récipient tel un ballon tricol, pour 1 équivalent molaire de substrat de formule Il, de 0,05 à 0,01 équivalent molaire de nitrate de cuivre" ou de chlorure ferrique et de 75 à 80 % en poids de solvant de type nitrile par rapport au poids du substrat.

De préférence, on agite le mélange en le maintenant à la température voulue, le plus souvent à la température ambiante, notamment en saturant l'atmosphère réactionnelle en oxygène par un barbotage ou une pression de 10,13 10⁴ Pa à 50,65 10⁴ Pa (1 à 5 atmosphères).

On dose alors dans le milieu réactionnel le composé α,β-dicarbonylé acyclique formé. Ce produit peut être isolé et purifié par des méthodes connues en soi, et explicitées dans les exemples illustrant la présente invention.

Parmi les composés α,β-dicarbonylés acycliques de formule I accessibles par le procédé de la présente invention, on peut citer :
- l'α-cétohexanoate d'éthyle
- l'α-cétobutyrate d'éthyle
- le phénylpyruvate d'éthyle
- le pyruvate d'éthyle
- le phénylglyoxylate d'éthyle
- le butane-2,3-dione
- le mésoxalate de diéthyle

La présente demande a aussi pour objet l'application du procédé ci-dessus à l'obtention des composés α,β-dicarbonylés acycliques de formule (I) précitée et notamment ceux ci-dessus.

Les exemples suivants, permettront de mieux comprendre la présente invention.

### EXEMPLE 1

Dans un tricol de 125 ml, muni d'un thermomètre, d'un agitation magnétique, d'un tube amenant l'oxygène et d'un réfrigérant surmonté d'un bulleur, on introduit :
- 24,7 g (0,13 mol., 1 éq.) de 2-n butylacétoacétate d'éthyle (pureté 97 %)
- 0,3204 g (1,3 mmoles, 0,01 éq.) de nitrate de cuivre ^{II} trihydraté (pureté 99 % min)
- et 100 g d'acétonitrile.

On agite le mélange sous barbotage d'oxygène (100 ml/mn) en maintenant une température de 20-25°C avec un bain d'eau.

On évapore sous pression réduite le solvant et l'acide acétique formés. Puis on ajoute 100 ml d'acétate d'éthyle et 30 ml d'eau. On agite et on sépare les phases. On lave la phase organique avec 10 ml d'eau. Puis on évapore sous pression réduite l'acétate d'éthyle. On distille les 23 g de produit brut obtenu. On récupère 17 g d'α-cétohexanoate d'éthyle (83 % de rendement) qui distille à 68°C /533,2 Pa (4 mmHg) (litt.84°C/1333 Pa (10 mmHg.)).

(La structure du produit a été confirmée par analyse RMN H¹ 200 MHz).

### EXEMPLE 2

Dans un tricol de 100 ml, muni d'un thermomètre, d'une agitation magnétique, d'un tube plongeant amenant l'oxygène, et d'un réfrigérant surmonté d'un bulleur, on introduit:
- 0,98 g (6 mmoles) de 2-éthylacétoacétate d'éthyle (pureté ∼ 97 %),
- 0,0476 g (0,3 mmole, 0,05 éq.) de chlorure ferrique (pureté ∼ 98 %), et
- 23,5 g d'acétonitrile.

On agite le mélange à 20°C sous barbotage d'oxygène (∼ 30 ml/mn.).

Après 6 heures de réaction on dose dans le milieu par HPLC (par étalonnage externe, après oximation par la méthoxylamine) 0,78 g d'α-cétobutyrate d'éthyle soit un rendement de 80 %.

### EXEMPLE 3

Dans un ballon tricol de 500 ml muni d'un thermomètre, d'une agitation magnétique, d'un tube plongeant, d'un réfrigérant surmonté d'un bulleur, on introduit :
- 33 g de 2-benzylacétoacétate d'éthyle (pureté 98 % min) (0,147 moles),
- 1,80 g de nitrate de cuivre trihydraté (pureté 99 % min) (7,45 mmoles), et
- 300 g d'acétonitrile.

On maintient 6 heures à 20°C sous agitation et barbotage d'oxygène (60 ml/mn).

On additionne 10 g de résine IRC 718 (Rohm et Haas). On maintient 1 h sous agitation à température ambiante, puis on filtre la résine qu'on lave sur le filtre avec un peu d'acétonitrile. On concentre le milieu à 40°C au rotavapor sous la pression réduite de la trompe à eau, puis on distille le produit brut obtenu. On recueille 2,5 g de benzaldéhyde (Téb = 55°C sous 266,6 Pa (2 mm de Hg)), puis 7,5 g d'une huile jaune qui distille à 110-115°C sous 133,3 Pa (1 mm de Hg) (lit.149-151°C sous 1999,5 Pa (15 mm de Hg)) qui est identifié par RMN comme étant le phénylpyruvate d'éthyle contenant une petite quantité de forme énol, structure confirmée par RMN H¹ 200 Mz. Le rendement en phénylpyruvate d'éthyle est de 27 % par rapport au produit de départ.

### EXEMPLE 4

Dans un ballon tricol de 100 ml muni d'un thermomètre, d'une agitation mécanique, d'un réfrigérant surmonté d'un bulleur, on introduit :
- 2,80 g de méthylmalonate de diéthyle (pureté 99 % ) (16 mmoles),
- 0,194 mg de nitrate de cuivre ^{II} trihydraté (pureté 99 % min) (0,8 mmole), et
- 32,2 g d'acétonitrile.

On maintient 16 h sous agitation et barbotage d'oxygène (30 ml/mn). On dose par HPLC (par étalonnage externe après oximation par la méthoxylamine) 1,74 g de pyruvate d'éthyle, soit un rendement de 93,5 %.

### EXEMPLE 5

Dans un tricol de 100 ml muni d'un thermomètre, d'une agitation magnétique, d'un tube plongeant amenant l'oxygène et d'un réfrigérant surmonté d'un bulleur, on introduit :
- 2,8 g (13 mmoles) de diéthyl-2oxalylbutyrate brut (synthétisé selon Organic Synthesis Coll. Vol. Il, 272-273),
- 0,151 g (0,65 mmole) de nitrate de cuivre Il trihydraté (pureté 99 % min.), et
- 32,2 g d'acétonitrile.

On maintient sous agitation et barbotage d'oxygène pendant 16 heures à T = 20°C. On dose dans le milieu réactionnel par HPLC (par étalonnage externe après oximation avec la méthoxylamine) 1,44 g d'α-cétobutyrate d'éthyle soit un rendement de 84,5 %.

### EXEMPLE 6

Dans un ballon tricol de 500 ml muni d'un thermomètre, d'une agitation mécanique et d'un tube plongeant pour amener l'oxygène, on introduit :
- 50 g de phényl malonate de diéthyle (pureté 98 %) (0,207 mole),
- 2,55 g de nitrate de cuivre (pureté 99 % min) (10,5 mmoles), et
- 450 g d'acétonitrile.

On maintient sous agitation et sous barbotage d'oxygène (60 ml/mn) pendant 12 h à une température de 20°C. On additionne au milieu réactionnel 20 g de résine IRC 718 (Rohm et Haas). On maintient 1 h sous agitation à température ambiante. On filtre la résine qu'on lave avec un peu d'acétonitrile. Le filtrat est concentré au rotavapor sous la pression réduite de la trompe à eau, puis distillé. On recueille la fraction qui distille à 95-100°C sous 133,3 Pa (1 mm de Hg) (lit. 80°C sous 26,7 Pa (0,2 mmHg)) soit 27,5 g qui correspond à un rendement de 74,6 %.

La structure du phénylglyoxylate d'éthyle a été confirmée par analyse RMN H¹ 200 Mz.

### EXEMPLE 7

Dans un tricol de 100 ml, muni d'un thermomètre, d'une agitation magnétique, d'une arrivée de gaz et d'un réfrigérant surmonté d'un bulleur, on introduit :
- 1,1571 g (9,7 mmoles) de 3-méthylacétylacétone (technique)
- 0,1188 g (0,48 mmole) de nitrate de cuivre ^{II} trihydraté (pureté 99 % min.), et
- 23,5 g d'acétonitrile.

On abaisse la température du mélange à 5 - 10°C avec un bain de glace. On agite sous barbotage d'oxygène (30 ml/mn). On dose, dans le milieu réactionnel par HPLC (par étalonnage externe, après oximation par la méthoxylamine) au bout de 8 h, 0,57 g de butan-2,3-dione (68 % de rendement).

### EXEMPLE 8

Dans un tricol de 100 ml, muni d'un thermomètre, d'une agitation magnétique, d'un tube plongeant amenant l'oxygène et d'un réfrigérant surmonté d'un bulleur on introduit :
- 0,656 g de 2-acétylmalonate de diéthyle techn. (3,24 mmoles),
- 0,0466 g de nitrate de cuivre ^{II} trihydraté (pureté 99 % min) (0,188 mmole), et
- 17 g d'acétonitrile.

On maintient sous agitation et barbotage d'oxygène (∼ 30 ml/mn), à 20 °C pendant 16 heures. On additionne 1 g de résine IRC 718 (Rohm et Haas). On maintient 1 heure sous agitation à température ambiante. On filtre la résine qu'on lave avec un peu d'acétonitrile. Dans le filtrat (18,7 g) on dose par CPG capillaire (étalonnage interne ,étalon oxalate de diéthyle), 0,20 g de mésoxalate de diéthyle, soit un rendement de 32,5 %.

### EXEMPLE DE COMPARAISON 1

Dans un tricol de 100 ml, muni d'un thermomètre, d'une agitation magnétique, d'un tube plongeant amenant l'oxygène, et d'un réfrigérant surmonté d'un bulleur on introduit :
- 0,98 g (6 mmoles) de 2-éthylacétoacétate d'éthyle (pureté - 97 %),
- du catalyseur à raison de 0,05 éq, et
- 23,5 g d'acétonitrile.

On agite le mélange à 20°C sous barbotage d'oxygène (∼ 30 ml/mn).

Après 2 et 4 heures de réaction on dose l'α -cétobutyrate d'éthyle formé dans le milieu par HPLC (étalonnage externe après oximation avec la méthoxylamine).

Les résultats sont consignés dans le tableau I suivant :

**TABLEAU I**

| | | | **Rdt (%)∗** | |
|---|---|---|---|---|
| **Essai** | **Catalyseurs** | **Solubilité** | **2 h** | **4 h** |
| 1 | Cu(NO₃)₂, 3H₂O | soluble | 75 | > 95 |
| 2 | Cu(ClO₄)₂, 6H₂O | soluble | 8 | 27 |
| 3 | Cu(OAc)₂,4H₂O | soluble | 3 | 4 |
| 4 | CuCl₂,2H₂O | soluble | 3 | 7 |
| 5 | CuCl₂ | soluble | 2 | 5 |
| 6 | Cu(SO₄)₂,5H₂O | peu soluble | 3 | 4 |
| 7 | CuBr₂ | soluble | 0 | 0 |
| 8 | CuCI | soluble | 0 | 4 |
| 9 | CuCO₂,Cu(OH)₂ | peu soluble | 0 | 0 |
| 10 | Cu acétylacétonate | peu soluble | 0 | 0 |
| 11 | Cu éthylacétoacétate | peu soluble | 0 | 0 |
| 12 | FeCl₃ | soluble | 65 | 85 |
| 13 | Fe(NO₃)₃,9H₂O | soluble | 24 | 27 |
| 14 | Co(OAc)₂,4H₂O | peu soluble | 1 | 4 |
| 15 | CoCl₂,6H₂O | soluble | 7 | 13 |
| 16 | Co(NO₃)₂,6H₂O | soluble | 0 | 0 |
| 17 | Co(SO₄)₂,7H₂O | peu soluble | 0 | 0 |
| 18 | Mn(OAc)₂,H₂O | peu soluble | 4 | 9 |
| 19 | MnCl₂,4H₂O | peu soluble | 2 | 3 |
| 20 | Mn(NO₃)₂,6H₂O | soluble | 0 | 0 |

| | | | | |
|---|---|---|---|---|
| ∗ *déterminé par dosage HPLC dans le milieu réactionnel (par étalonnage externe après oximation avec la méthoxylamine)* | | | | |

L'analyse de ces résultats fait apparaître clairement que le nitrate de Cu^{II} (essai 1) et que le chlorure ferrique (essai 12) sont de loin les catalyseurs les plus efficaces par rapport à tous les autres catalyseurs testés.

### EXEMPLE DE COMPARAISON 2

Dans un tricol de 100 ml, muni d'un thermomètre, d'une agitation magnétique, d'une arrivée de gaz et d'un réfrigérant surmonté d'un bulleur, on pèse :
- 0,98 g (6 mmoles, 1 éq.) de 2-éthylacétoacétate d'éthyle (pureté ∼ 97 %)

On ajoute 0,0732 g (0,3 mmole 0,05 éq.) de nitrate de cuivre^{II} trihydraté (pureté 99 % min) dissous dans divers solvants de type nitrile (concentration massique du substrat : 4 % par rapport au solvant).

On agite le mélange en maintenant la température à 20°C avec un bain d'eau sous barbotage d'oxygène (30 ml/mm). On dose, dans le milieu réactionnel l'α-cétobutyrate d'éthyle formé en fonction du temps de réaction par HPLC (par étalonnage externe après oximation avec la méthoxylamine).

Les résultats sont consignés dans le tableau Il suivant :

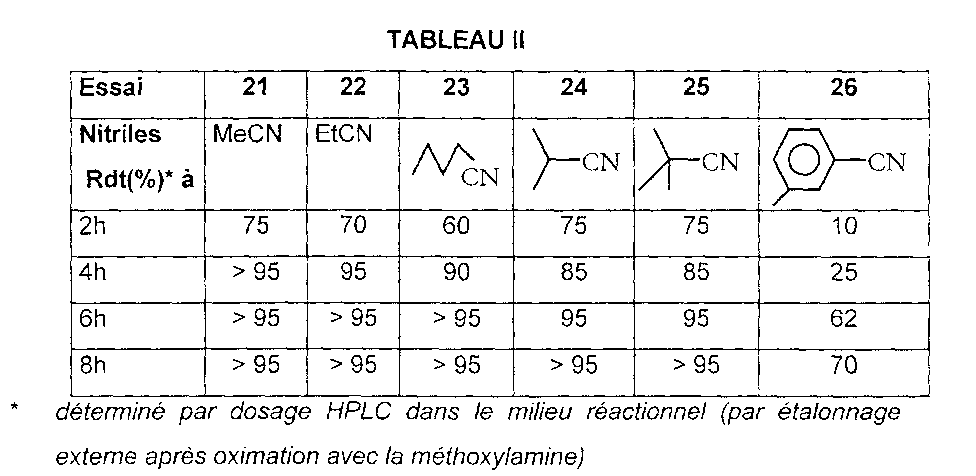

Le nitrate de Cu^{II} possède sensiblement la même réactivité dans les différents nitriles aliphatiques. Elle est légèrement inférieure dans les nitriles aromatiques.

### EXEMPLE DE COMPARAISON 3

On a étudié l'efficacité des catalyseurs Cu(NO₃)₂,3H₂O et FeCl₃ dans divers solvants de type polaires, apolaires, protiques, aprotiques et dans des mélanges de solvants.

Dans un tricol de 100 ml, muni d'un thermomètre, d'une agitation magnétique, d'un tube plongeant on introduit :
- 0,98 g (6 mmoles) de 2-éthylacétoacétate d'éthyle (pureté ∼ 97 %),
- du catalyseur Cu(NO₃)₂,3H₂O ou FeCl₃ à raison de 0,05 éq. et divers solvants ou mélanges de solvants.

On agite le mélange à 20°C sous barbotage d'oxygène (∼ 30 ml/mn).

Après 2 et 4 heures de réaction, on dose I' α-cétobutyrate d'éthyle formé dans le milieu par HPLC (par étalonnage externe après oximation avec la méthoxylamine).

Les résultats sont consignés dans le tableau III suivant :

**TABLEAU III**

| | | | **Rdt (%)∗** | |
|---|---|---|---|---|
| **Essai** | **Catalyseurs** | **Solvant** | **2 h** | **4 h** |
| 21 | Cu(NO₃)₂, 3H₂O | acétonitrile | 75 | > 95 |
| 27 | Cu(NO₃)₂, 3H₂O | acide acétique | 0 | 0 |
| 28 | Cu(NO₃)₂, 3H₂O | éthanol | 0 | 0 |
| 29 | Cu(NO₃)₂, 3H₂O | acétate d'éthyle | 0 | 0 |
| 30 | Cu(NO₃)₂, 3H₂O | diméthylformamide | 0 | 0 |
| 31 | Cu(NO₃)₂, 3H₂O | toluène | 0 | 0 |
| 32 | FeCl₃ | acétonitrile | 65 | 85 |
| 33 | FeCl₃ | acide acétique | 0 | 0 |
| 34 | FeCl₃ | éthanol | 0 | 0 |
| 35 | FeCl₃ | acétate d'éthyle | 0 | 0 |
| 36 | Cu(NO₃)₂, 3H₂O | MeCN/eau(75-25) | 0 | 0 |
| 37 | Cu(NO₃)₂, 3H₂O | MeCN/eau (95-5) | 19 | 33 |
| 38 | Cu(NO₃)₂, 3H₂O | MeCN/toluène (30-70) | 35 | 47 |
| 39 | Cu(NO₃)₂, 3H₂O | MeCN/toluène (5-95) | 0 | 0 |
| 40 | Cu(NO₃)₂, 3H₂O | MeCN/MeCOOEt (5-95) | 0 | 0 |
| 41 | Cu(NO₃)₂, 3H₂O | MeCN/MeCOOEt (10-90) | 15 | 28 |
| 42 | Cu(NO₃)₂, 3H₂O | MeCN/MeCOOEt (20-80) | 45 | 65 |

| | | | | |
|---|---|---|---|---|
| ∗ *déterminé par dosage HPLC dans le milieu réactionnel (par étalonnage externe après oximation avec la méthoxylamine)* | | | | |

Le nitrate de cuivre^{II} et le chlorure ferrique ne présentent d'activité catalytique que dans l'acétonitrile. Ils n'ont aucune activité dans les autres solvants, notamment dans l'acide acétique et l'éthanol.

Ces résultats montrent de plus que la réaction peut être effectuée dans des mélanges de solvants. Cependant, la présence d'acétonitrile est indispensable, et la rapidité de la réaction augmente quand la proportion d'acétonitrile augmente.

### EXEMPLE 9

On a aussi étudié l'efficacité de la quantité de catalyseur Cu(NO₃)₂,3H₂O sur la vitesse de la réaction.

Dans un tricol de 100 ml, muni d'un thermomètre, d'une agitation magnétique, d'un tube plongeant amenant l'oxygène, et d'un réfrigérant surmonté d'un bulleur, on introduit :
- 0,98 g (6 mmoles) de 2-éthylacétoacétate d'éthyle (pureté ∼ 97 %),
- du catalyseur Cu(NO₃)₂,3H₂O à raison de 0,05 éq. à 0,001 éq., et
- 23,5 g d'acétonitrile.

On agite le mélange à 20°C sous barbotage d'oxygène (∼ 30 ml/mn).

Après 2 et 4 heures de réaction on dose I' α-cétobutyrate d'éthyle formé dans le milieu par HPLC (par étalonnage externe après oximation avec la méthoxylamine).

Les résultats sont consignés dans le tableau IV suivant :

**TABLEAU IV**

| Essai | Concentration massique en β-cétoester (%) | Quantité de catalyseur (% molaire) | température de réaction | Rdt (%)∗ | | | |
|---|---|---|---|---|---|---|---|
| | | | | 2h | 4h | 6h | 8h |
| 43 | 4 | 5 | ambiante | 75 | > 95 | > 95 | > 95 |
| 44 | 8 | 5 | ambiante | 90 | > 95 | > 95 | > 95 |
| 45 | 4 | 1 | ambiante | 31 | 71 | 95 | > 95 |
| 46 | 12 | 1 | ambiante | 71 | 81 | 91 | > 95 |
| 47 | 20 | 1 | ambiante | 41 | 82 | 95 | > 95 |
| 48 | 30 | 1 | ambiante | 46 | 57 | 66 | 73 |
| 49 | 90 | 2 | ambiante | 17 | 21 | 34 | 48 |
| 50 | 20 | 0,1 | 50°C | / | / | > 95 | > 95 |

| | | | | | | | |
|---|---|---|---|---|---|---|---|
| ∗ *déterminé par dosage HPLC dans le milieu réactionnel (par étalonnage externe, après oximation avec la méthoxylamine).* | | | | | | | |

La réaction est favorisée par l'augmentation de la quantité de sel métallique. Une température plus élevée permet de diminuer cette quantité. La quantité de nitrile est également importante. Une concentration de 20 à 25 % de substrat dans le mélange est préférée.

### EXEMPLE 10

Dans un tricol de 100 ml, muni d'un thermomètre, d'une agitation magnétique, d'une arrivée de gaz et d'un réfrigérant surmonté d'un bulleur, on introduit :
- 0,98 g (6 mmoles, 1 éq.) de 2-éthylacétoacétate d'éthyle (pureté ∼ 97 %)
- 0,0732 g (0,3 mmole 0,05 éq.) de nitrate de cuivre ^{II} trihydraté (pureté 99 % min), et
- 23,5 g d'acétonitrile.

On agite le mélange en maintenant la température à 20°C avec un bain d'eau sous barbotage d'air (85 ml/mn). On dose, dans le milieu réactionnel au bout de 4 h par HPLC (par étalonnage externe après oximation avec la méthyloxylamine) 0,78 g d' α-cétobutyrate d'éthyle (rendement > 95 %).

### EXEMPLE 11

Dans un autoclave de 2 l en verre, muni d'une agitation mécanique, d'une arrivée de gaz, d'un thermomètre et d'un chauffage par circulation d'huile dans une double enveloppe, on introduit :
- 135 g (0,85 mol, 1 éq.) de 2-éthylacétoacétate d'éthyle
- 2 g (0,0085 mol, 0,01 éq.) de nitrate de cuivre" trihydraté,
- 500 g d'acétonitrile.

On agite le mélange en maintenant toujours la température inférieure à 40°C et on charge le réacteur sous 5 bars d'oxygène. Dès que la pression devient inférieure à 2 bars ,on recharge en oxygène (5 bars) et ainsi de suite jusqu'à ce que la consommation en oxygène soit négligeable (soit environ 6 heures).

On distille ensuite sous pression réduite le milieu et on obtient 77 g d' α-cétobutyrate d'éthyle soit un rendement d'environ 70 % (par CPG directe).

## Revendications

1. Procédé de préparation de composés α,β-dicarbonylés acycliques de formule (I) 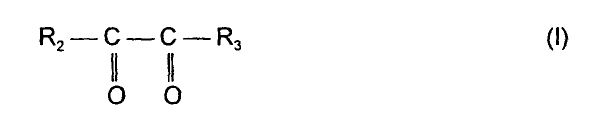 dans laquelle R₂ représente un radical alkyl renfermant de 1 à 6 atomes de carbone, ou un radical phényl ou un radical benzyl pouvant être diversement substitué sur le cycle par des substituants alkyl renfermant de 1 à 6 atomes de carbone, par des substituants alkoxy renfermant de 1 à 4 atomes de carbone ou par des substituants halogènes, ou un radical ester carboxylique renfermant de 2 à 5 atomes de carbone et R₃ représente un radical alkyl renfermant de 1 à 4 atomes de carbone, ou un radical alkoxy renfermant de 1 à 4 atomes de carbone ou un radical ester carboxylique renfermant de 2 à 5 atomes de carbone, **caractérisé par** le fait que l'on fait réagir dans un solvant de type nitrile un composé α,γ dicarbonylé acyclique de formule (II) 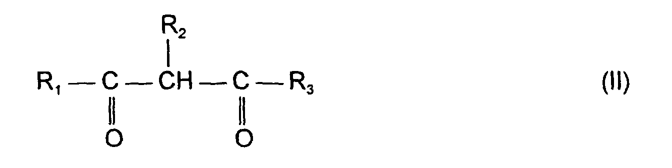 dans laquelle R₁ représente un radical alkyl renfermant de 1 à 6 atomes de carbone ou un radical éthoxy, ou un radical α-cétocarboxylate d'alkyle renfermant de 3 à 6 atomes de carbone ou un radical ester carboxylique renfermant de 2 à 5 atomes de carbone et R₂ et R₃ ont les mêmes significations que précédemment, avec de l'oxygène moléculaire en présence de nitrate de cuivre^{II} ou de chlorure ferrique comme catalyseur.

2. Procédé selon la revendication 1, **caractérisé par** le fait que le solvant de la réaction est un solvant de type nitrile aliphatique ou aromatique.

3. Procédé selon la revendication 2, **caractérisé par** le fait que le solvant de la réaction est du type nitrile aliphatique.

4. Procédé selon l'une des revendications 1 à 3, **caractérisé par** le fait que la quantité de nitrate de cuivre" ou de chlorure ferrique est comprise entre 0,1 équivalent molaire et 0,001 équivalent molaire par rapport au substrat de départ de formule (II).

5. Procédé selon la revendication 4, **caractérisé par** le fait que la quantité de nitrate de cuivre" ou de chlorure ferrique est comprise entre 0,05 équivalent molaire et 0,01 équivalent molaire par rapport au substrat de départ de formule générale (II).

6. Procédé selon l'une des revendications 1 à 5, **caractérisé par** le fait que la réaction est effectuée en milieu saturé en oxygène réalisé par un barbotage d'oxygène ou d'air dans le mélange réactionnel.

7. Procédé selon l'une des revendications 1 à 5, **caractérisé par** le fait que la réaction est effectuée en milieu saturé en oxygène réalisé sous pression d'oxygène de 10,13 10⁴ Pa à 50,65 10⁴ Pa (1 à 5 atmosphères).

8. Procédé selon la revendication 6 ou selon la revendication 7, **caractérisé par** le fait que l'oxygène utilisé provient d'un débit d'air ou d'une pression d'air.

9. Application du procédé selon l'une des revendications 1 à 8 à l'obtention de l'α-cétobutyrate d'éthyle, du phénylpyruvate d'éthyle, du pyruvate d'éthyle, du phénylglyoxylate d'éthyle, de la butane-2,3-dione ou du mésoxalate de diéthyle.

## Claims

1. Preparation process for acyclic α,β-dicarbonylated compounds of formula (I) 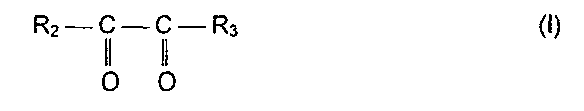 in which R₂ represents an alkyl radical containing 1 to 6 carbon atoms, or a phenyl radical or a benzyl radical being able to be substituted in various ways on the cycle by alkyl substituents containing 1 to 6 carbon atoms, by alkoxy substituents containing 1 to 4 carbon atoms or by halogen substituents, or a carboxylic ester radical containing 2 to 5 carbon atoms, and R₃ represents an alkyl radical containing 1 to 4 carbon atoms, or an alkoxy radical containing 1 to 4 carbon atoms, or a carboxylic ester radical containing 2 to 5 carbon atoms, **characterized in that** an acyclic α,γ dicarbonylated compound of formula (II) 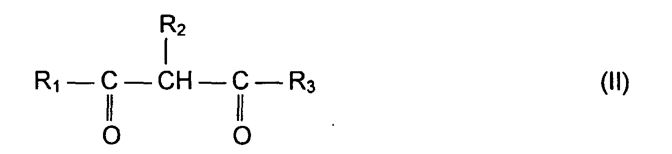 in which R₁ represents an alkyl radical containing 1 to 6 carbon atoms or an ethoxy radical, or an alkyl α-ketocarboxylate radical containing 3 to 6 carbon atoms or a carboxylic ester radical containing 2 to 5 carbon atoms, and R₂ and R₃ have the same meanings as previously is reacted in a nitrite-type solvent, with molecular oxygen in the presence of copper^{II} nitrate or ferric chloride as catalyst.

2. Process according to claim 1, **characterized in that** the reaction solvent is an aliphatic or aromatic-nitrile type solvent.

3. Process according to claim 2, **characterized in that** the reaction solvent is of the aliphatic-nitrile type.

4. Process according to one of claims 1 to 3, **characterized in that** the quantity of copper^{II} nitrate or ferric chloride is comprised between 0.1 molar equivalent and 0.001 molar equivalent with respect to the starting substrate of formula (II).

5. Process according to claim 4, **characterized in that** the quantity of copper^{II} nitrate or ferric chloride is comprised between 0.05 molar equivalent and 0.01 molar equivalent with respect to the starting substrate of general formula (II).

6. Process according to one of claims 1 to 5, **characterized in that** the reaction is carried out in a medium saturated in oxygen achieved by bubbling oxygen or air through the reaction mixture.

7. Process according to one of claims 1 to 5, **characterized in that** the reaction is carried out in a medium saturated in oxygen achieved under an oxygen pressure of 10.13 10⁴ Pa to 50.65 10⁴ Pa (1 to 5 atmospheres).

8. Process according to claim 6 or according to claim 7, **characterized in that** the oxygen used comes from an air supply or under an air pressure.

9. Use of the process according to one of claims 1 to 8 for obtaining ethyl α-ketobutyrate, ethyl phenylpyruvate, ethyl pyruvate, ethyl phenylglyoxylate, butane-2,3-dione or diethyl mesoxalate.

## Patentansprüche

1. Verfahren zur Herstellung von acyclischen *α*,*β*-Dicarbonylverbindungen der Formel (I) 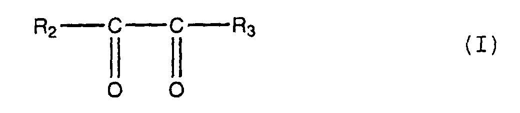 worin R₂ einen Alkylrest mit 1 bis 6 Kohlenstoffatomen oder einen Phenylrest oder einen Benzylrest darstellt, die am Ring unterschiedlich substituiert sein können mit Alkyl-Substituenten mit 1 bis 6 Kohlenstoffatomen, mit Alkoxy-Substituenten mit 1 bis 4 Kohlenstoffatomen oder mit Halogen-Substituenten oder einem Carboxylesterrest mit 2 bis 5 Kohlenstoffatomen und R₃ einen Alkylrest darstellt mit 1 bis 4 Kohlenstoffatomen oder einen Alkoxyrest mit 1 bis 4 Kohlenstoffatomen oder einen Carboxylesterrest mit 2 bis 5 Kohlenstoffatomen, **dadurch gekennzeichnet, daß** man in einem Nitrillösungsmittel eine acyclische α,γ-Dicarbonylverbindung der Formel (II) 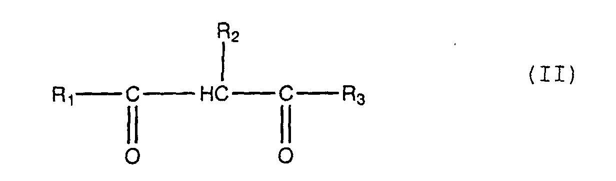 worin R₁ einen Alkylrest mit 1 bis 6 Kohlenstoffatomen oder einen Ethoxyrest oder einen α-Cetocarboxylatalkylrest mit 3 bis 6 Kohlenstoffatomen oder einen Carboxylesterrest mit 2 bis 5 Kohlenstoffatomen darstellt und R₂ und R₃ dieselbe Bedeutung wie zuvor haben, mit molekularem Sauerstoff in Gegenwart von Kupfer(II)-nitrat oder Eisenchlorid als Katalysator umsetzt.

2. Verfahren nach Anspruch 1, **dadurch gekennzeichnet, daß** das Reaktionslösungsmittel ein aliphatisches oder aromatisches Nitrillösungsmittel darstellt.

3. Verfahren nach Anspruch 2, **dadurch gekennzeichnet, daß** das Reaktionslösungsmittel ein aliphatisches Nitril darstellt.

4. Verfahren nach mindestens einem der Ansprüche 1 bis 3, **dadurch gekennzeichnet, daß** die Menge an Kupfer(II)-nitrat oder Eisenchlorid zwischen 0,1 Mol-Äquivalenten und 0,001 Mol-Äquivalenten, bezogen auf das Ausgangssubstrat der Formel (II), beträgt.

5. Verfahren nach Anspruch 4, **dadurch gekennzeichnet, daß** die Menge an Kupfer(II)-nitrat oder Eisenchlorid zwischen 0,05 Mol-Äquivalenten und 0,01 Mol-Äquivalenten, bezogen auf das Ausgangssubstrat der allgemeinen Formel (II), beträgt.

6. Verfahren nach mindestens einem der Ansprüche 1 bis 5, **dadurch gekennzeichnet, daß** die Umsetzung in einem gesättigten Sauerstoffmilieu durchgeführt wird, verwirklicht durch ein Durchblasen von Sauerstoff oder Luft durch die Reaktionsmischung.

7. Verfahren nach mindestens einem der Ansprüche 1 bis 5, **dadurch gekennzeichnet, daß** die Reaktion in einem gesättigten Sauerstoffmilieu durchgeführt wird, verwirklicht durch einen Sauerstoffdruck von 10,13 x 10⁴ Pa bis 50,65 x 10⁴ Pa (1 bis 5 Atmosphären).

8. Verfahren nach Anspruch 6 oder Anspruch 7, **dadurch gekennzeichnet, daß** der eingesetzte Sauerstoff aus einem Luftstrom oder aus Druckluft herrührt.

9. Anwendung des Verfahrens nach mindestens einem der Ansprüche 1 bis 8 zur Gewinnung von Ethyl-α-cetobutyrat, Ethylphenylpyruvat, Ethylpyruvat, Ethylphenylglyoxylat, Butan-2,3-dion oder Diethylmesoxalat.
